(19) Europäisches Patentamt
European Patent Office
Office européen des brevets

(11) **EP 4 524 816 A1**

(12) **EUROPEAN PATENT APPLICATION**

(43) Date of publication:
**19.03.2025 Bulletin 2025/12**

(21) Application number: **23315347.7**

(22) Date of filing: **12.09.2023**

(51) International Patent Classification (IPC):
**G06N 3/045** $^{(2023.01)}$   **G06N 3/047** $^{(2023.01)}$
**G06N 3/09** $^{(2023.01)}$   **G06N 3/096** $^{(2023.01)}$
**G06N 7/01** $^{(2023.01)}$

(52) Cooperative Patent Classification (CPC):
**G06N 7/01; G06N 3/045; G06N 3/047; G06N 3/09;
G06N 3/096**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **SUPERSONIC IMAGINE**
**13290 Aix-en-Provence (FR)**

(72) Inventor: **ZHANG, Bo**
**13109 Simiane-Collongue (FR)**

(74) Representative: **Paustian & Partner**
**Patentanwälte mbB**
**Oberanger 32**
**80331 München (DE)**

(54) **A METHOD OF QUANTIFYING A POSTERIOR CONFIDENCE**

(57)    The invention relates to a to a computer-implemented method, comprising: quantifying a posterior confidence of an output of a layer of an artificial intelligence (AI) model for a set of input data samples using a mixture model, the AI model being a classifier model.

100

| S1 (optional): providing an artificial intelligence (AI) model |
| --- |

↓

| S2 (optional): inputting into the AI model a set of input data |
| --- |

↓

| S3: quantifying a model likelihood of outputs of a layer of the model for the set of input data samples using a mixture model |
| --- |

↓

| S4 (optional): providing an assumed prevalence prior on a class distribution |
| --- |

↓

| S5: (optional): calibrating the quantified model likelihood |
| --- |

↓

| S6 (optional): estimating a class probability of an input data sample using the quantified (and optionally calibrated) model likelihood |
| --- |

Fig. 1

**Description**

BACKGROUND

Artificial intelligence (AI) is the ability of machines to perform tasks that are typically associated with human intelligence, such as learning and problem-solving.

AI algorithms or models can have various applications, for example in the field of computer vision. A more specific example concerns image recognition or classification in the field of medical imaging. For instance, an AI model may estimate a breast lesion malignancy probability, a Bi-RADS (Breast Imaging Reporting and Data System) lesion classification probability, a liver lesion classification probability, a TI-RADS (Thyroid Imaging Reporting & Data System) classification probability of a thyroid, etc.

A part of AI includes Machine learning which solves problems by helping machines 'discover' their 'own' algorithms, without needing to be explicitly told what to do by any human-developed algorithms. These algorithms or neural networks may also be generally referred to as models.

Deep learning is part of a broader family of machine learning methods, which is based on artificial neural networks with representation learning. The adjective "deep" in deep learning refers to the use of multiple layers in the network. Methods used can be either supervised, semi-supervised or unsupervised. Exemplary supervised learning tasks comprise classification and regression.

The word "deep" in "deep learning" refers to the number of layers through which the data is transformed. For example, a multilayer network may comprise an input layer, one or several hidden layers and an output layer.

Goodfellow et.al. provides an introduction to a broad range of topics in deep learning, covering mathematical and conceptual background, deep learning techniques used in industry, and research perspectives, cf. Goodfellow, Ian, Yoshua Bengio, and Aaron Courville. Deep Learning. Cambridge, MA: MIT Press, 2016.

In case of for example a classification task, the output layer of a classification model may conventionally rely on a softmax logistic function to estimate an artificial probability of a prediction. Accordingly, the classification model may output an estimated class, together with a probability. For example, in the medical domain a model may receive a medical image and classify the Bi-RADS level of a detected breast lesion and its probability.

However, it may appear unclear for a user, such as an examining person, for example a doctor, physicist or radiologist, how to interpret this probability of an estimated class.

In particular, it may be unclear, whether the probability is a pure confidence of the AI model or whether contextual uncertainty related to a particular use case should be considered when interpreting the probability. For example, contextual uncertainty may be caused by the circumstance that -depending on the particular use case (for instance screening or diagnostics)- the background population may differ. According to another example, contextual uncertainty may be caused by the circumstance that - depending on the particular use case- a patient to be examined may be from a specific group, for instance conditioned by lesion history, ethnicity, etc.

In view of the above, there is a need to solve such drawbacks and associated disadvantages.

SUMMARY OF THE DISCLOSURE

A computer-implemented method, comprising:

quantifying a posterior confidence of an output of a layer of an artificial intelligence (AI) model for a set of input data samples using a mixture model, the AI model being a classifier model.

In other words, the method may comprise: providing an artificial intelligence (AI) model configured to classify input data, and quantifying a posterior confidence of an output of a layer of the model for a set of input data samples using a mixture model.

By providing such a method, it becomes possible to calibrate a more meaningful class probability for an AI model without requiring new training data. In particular, the output probability (i.e. the quantified posterior confidence according to the present disclosure) can be better calibrated than conventional SoftMax based artificial logistic probability.

Conventional AI models (for example deep neural networks) rely on a softmax logistic function to estimate an artificial probability of a prediction, for example for classifying input data. This probability is suboptimal if for example the encoded features are heteroskedastic. It is also ignorant of a prior probability of classes.

In contrast, according to the present disclosure, for example a gaussian mixture based parametric model may be used to quantify the posterior confidence. This may then be taken into account in a Bayesian framework to better calibrate the network's probability estimation.

This may be done on an existing network without re-retraining. In other words, there is no need to retrain the initial model. It is advantageously sufficient to replace the output layer, as described above. Hence, the method is computationally significantly less expensive compared to any conventional methods which require training a model.

When using a conventional AI model, it may be unclear, how to interpret a probability of an estimated class, as determined by the AI model. This deficiency may be at least partially due to limitations in the training data used for training the AI model. For example, in the medical domain the training data may consist of medical images and respective classification labels. These may be generated by an annotator (for example a doctor, physicist, radiologist or other specialist) who annotates each image with a class. However, such annotations typically do not contain any confidence or probability information regarding a contextual uncertainty. This deficiency is even more critical, since it is usually difficult to obtain many training data in the medical domain.

In a more specific example, a single annotator may annotate a medical image with a Bi-RADS level without adding a probability or confidence to said selected level. As a result, one particular Bi-RADS level may be annotated as being the correct output with a probability of 100%, meanwhile each other possible Bi-RADS level may be annotated as being the correct output with a probability of 0%. Accordingly, the training data comprise no ambiguity in the training labels. Hence, the resulting training data typically lead to an overrated confidence of a trained AI model. In other words, the probability of a conventional trained model is often too high.

A conventional softmax function cannot overcome such deficits, mainly because a confidence information is inexistant in the labels. In addition, the softmax function is usually oversimplistic, due to its piecewise linear decision boundary.. For example, the softmax does not suitably consider a mixture distribution of heteroscedastic gaussians of the encoded features in a layer of the AI model, contrary to the AI model of the present disclosure. In particular, the AI model of the present disclosure may better exploit inherent data in the model (for example the neural network) is exploited, what can lead to a more realistic confidence or probability of the estimated classes.

As a further advantage the AI model of the present disclosure is not computationally significantly more expensive than a conventional AI model using a softmax function.

The operation of providing an artificial intelligence (AI) model may comprise inputting into the AI model a set of input data which in response estimates a class probability.

A class probability may be estimated among a plurality of predefined possible classes.

The "output of a layer" according to the present disclosure may comprise a plurality of outputs, for instance for each input a respective output. For example, the output per input may be a single value (i.e. a scalar), for instance in case the layer has a single neuron. In case the layer has a plurality of neurons, the output per input may be vector with a respective number of dimensions.

Quantifying a posterior confidence of an output of a layer may comprise or may mean modelling the output of the layer

The layer of the present disclosure may be a predefined layer.

The method of the present disclosure may be a method of quantifying a posterior confidence.

The set of input data may comprise a plurality of samples. A sample may be for example an image, in a more specific example a medical image, illustrating for example a representation of internal tissues of a human body.

Various classification tasks may be carried out, i.e. various types of class probabilities may be estimated. One example comprises classifying a lesion characteristic of tissue of a patient. For instance, the method may estimate a breast lesion malignancy probability, a Bi-RADS (Breast Imaging Reporting and Data System) lesion classification probability, a liver lesion classification probability, etc.

However, it is noted that the present disclosure is not limited to any specific kind of classification task or technical field. Also completely different tasks and technical fields are possible, for example classifying characteristics of a sound wave.

The layer may be at least one of:

a hidden layer of the model, e.g. the last hidden layer of the model,
an intermediate layer of the model,
a layer other than an output layer of the model, and
the layer preceding an output layer of the model, such as for example the last layer preceding an output layer of the model.

In other words, the AI model may comprise at least one hidden, i.e. intermediate layer.

For example, the model may comprise an optional input layer, one or several hidden or intermediate layers, and an output layer.

When referring to "an output of a layer", it may be referred to any layer of the model except the output layer.

The method may further comprise using the quantified posterior confidence as an output layer of the model.

The quantified posterior confidence may be expressed as or may constitute a mathematical function. This function may be used as the "new" or replacing output layer which replaces the initial output layer of the model. Thus, the "new" or replacing output layer may receive as inputs the outputs of the last hidden layer and calculates on this basis the outputs of the model.

The quantified posterior confidence may be configured to receive and process an output of the layer.

The method may further comprise estimating a class probability of an input data sample using the quantified posterior confidence.

A quantified posterior confidence function may be obtained by quantifying a posterior confidence of an output of a layer of the model for a set of input data samples using the mixture model.

The quantified posterior confidence may be a quantified posterior confidence function.

Accordingly, the "quantified posterior confidence" may be a mathematical function, which for example is able to calculate an output based on an input.

The method may further comprise:

providing an assumed prevalence prior on a class distribution, and
calibrating the quantified posterior confidence and/or the class probability of the input data sample based on the prevalence prior.

The class distribution may be a known or predetermined class distribution. According, the respective prior data may be provided to the method (for example a system on which the method is running) from an external device, or the data may be stored on the system.

Accordingly, a known class distribution may be used as a prior to calibrate the model and thus increase the accuracy of the model.

A Bayesian rule may be used for calibrating the quantified posterior confidence and/or the class probability of the input data sample.

The mixture model may be a probabilistic mixture model such as a Gaussian mixture model.

Generally the term "mixture model" may point out that there are several classes and hence not for example a single gaussian distribution.

Quantifying the posterior confidence of the layer may comprise: quantifying the probability distribution of the output of the layer, and/or recording mean and covariance statistics of the output of the layer for each possible output class of the model.

The model may be at least one of a machine learning model and a neural network. The model may also be or comprise a deep learning model, for example a neural network, such as a convolutional neural network (CNN).

The model may comprise at least one hidden layer and an output layer. For example, the at least one hidden layer may be the last or terminating layer z of the model whose output is inputted into the output layer of the model.

The model may be a first initial model comprising an initial output layer configured to perform a classification task.

In other words, the first, i.e. initial model, may be modified by replacing the last layer. This modified model may then be used in a final product, for example an imaging and/or classification system, as described in more detail below in context of the figures.

For example, the initial output layer may be configured to perform a K-class softmax rule. K may be at least 2. In other words, the initial output layer may be or may perform or may comprise a softmax logistic function.

The method may further comprise obtaining a first modified model by replacing the initial output layer of the first initial model by the quantified posterior confidence.

Accordingly, the first modified model may not use for example a K-class softmax rule anymore.

Advantageously, there is no need to retrain the initial model. It is sufficient to replace the output layer, as described above. Hence, the method is computationally significantly less expensive compared to any conventional methods which require training a model.

The present disclosure may further relate to a computer-implemented method of estimating a class probability of an input sample comprising estimating a class probability for the input sample using the first modified model.

The method may optionally comprise (for example before the operation of estimating a class probability) the operation of feeding an input sample to the first modified model.

The computer-implemented method of estimating a class probability may be used in or performed by a classification system as described above. The classification system may be part of or associated with for example a medical imaging device. The input sample may be for example a medical image. Various classification tasks may be carried out, i.e. various types of class probabilities may be estimated. One example comprises classifying a lesion characteristic of tissue of a patient. For instance, the method may estimate a breast lesion malignancy probability, a Bi-RADS (Breast Imaging Reporting and Data System) lesion classification probability, a liver lesion classification probability, etc.

However, it is noted that the present disclosure is not restricted to any specific kind of classification task or technical field. Also different tasks and technical fields are possible, for example classifying characteristics of a sound wave.

The method may further comprise selecting a prevalence prior as a function of the input sample and estimate a calibrated class probability for the input sample based on the prevalence prior using the first modified model.

The present disclosure may further relate to a method of generating training labels for an artificial intelligence (AI) algorithm, comprising:

applying any one of the methods described above to the set of input data samples to obtain a respective set of class probabilities, and generating the training labels based on the set of class probabilities.

Accordingly, the training dataset may comprise the set of input data and the associated labels.

Accordingly, other models may be trained, as further described below.

The present disclosure may further relate to a method of training an artificial intelligence (AI) model, comprising:

performing the method as described above to generate training labels,
training a first initial artificial intelligence (AI) model in a supervised manner using the training labels. The first initial artificial intelligence (AI) model may be the above-mentioned first initial artificial intelligence (AI) model or any other model.

For example, the set of input data may be used as input during training and the set of training labels may be used as target output.

Accordingly, the first initial artificial intelligence (AI) model may be trained (for example from scratch) on the training dataset. The first initial artificial intelligence (AI) model may be any kind of classification model. For example, it may comprise a conventional output layer, such as for instance a K-class softmax rule.

The method may further comprise obtaining a second artificial intelligence (AI) model by replacing the last layer of the trained first artificial intelligence (AI) model by the quantified posterior confidence,

running the second artificial intelligence (AI) model to obtain estimated class probabilities for the set of input data samples,
re-labelling the set of input data samples with the estimated class probabilities,
train a third artificial intelligence (AI) model based on the re-labelled set of input data samples.

The method may be iterated one or several times. Accordingly, the method may be further iteratively applied in the training phase, in order to advantageously calibrate the training and probability simultaneously.

For example, the method may further comprise obtaining a fourth artificial intelligence (AI) model by replacing the last layer of the trained third artificial intelligence (AI) model by the quantified posterior confidence,

running the fourth artificial intelligence (AI) model to obtain estimated class probabilities for the set of input data samples,
re-labelling the set of input data samples with the estimated class probabilities,
train a fifth artificial intelligence (AI) model based on the re-labelled set of input data samples.

In this way, both the training datasets, as well as the trained models may be successively ameliorated to represent the real classification problem more accurately.

The present disclosure may further relate to a computing device, comprising:

at least one processor, and
at least one memory storing computer-executable instructions, the computer-executable instructions when executed by the processor cause the computing device to perform a any one of the methods as described above.

The present disclosure may further relate to a computing device, comprising: at least one processor, and at least one memory storing computer-executable instructions, and an artificial intelligence (AI) model being a classifier model. The computer-executable instructions when executed by the processor comprise quantifying a posterior confidence of an output of a layer of the model for a set of input data samples using a mixture model.

The computing device may be configured to be associated with a display device, such that for example an image and an associated estimated class may be displayed on the display device.

The computing device may be configured to be associated with an input device configured to obtain one or several input samples and to provide it to the computing device.

The processor (or processing unit) may be a component of electronic devices that may be responsible for carrying out computational tasks. A processing unit may be or may comprise a Central Processing Unit (CPU), Graphics Processing Unit (GPU), Digital Signal Processor (DSP), Field-Programmable Gate Array (FPGA), and/or Application-Specific Integrated Circuit (ASIC). The method according to the present disclosure may also run on a virtual server.

The present disclosure may also relate to a system of classifying an input sample, the system comprising means for carrying out the method according to any examples of the present disclosure. For example, the system may comprise or may be a computing device, as described above.

The present disclosure may also relate to an imaging system (e.g., ultrasound imaging) comprising means for carrying out a method according to any examples of the present disclosure.

The present disclosure may also relate to a computer program comprising instructions which, when the program is executed by a computer, cause the computer to carry out the method according to any examples of the present disclosure.

The present disclosure may also relate to a computer-readable medium comprising instructions which, when executed by a computer, cause the computer to carry out the method according to any examples of the present disclosure.

It is intended that combinations of the above-described elements and those within the specification may be made, except where otherwise contradictory.

It is to be understood that both the foregoing general description and the following detailed description are exemplary and explanatory only, are provided for illustration purposes and are not restrictive of the disclosure, as claimed.

The accompanying drawings, which are incorporated in and constitute a part of this specification, illustrate examples of the disclosure and together with the description, and serve to support and illustrate the principles thereof.

BRIEF DESCRIPTION OF THE DRAWINGS

Fig. 1 schematically shows a method of quantifying a posterior confidence according to examples of the present disclosure.

Fig. 2 schematically shows an initial AI model and a first modified AI model according to examples of the present disclosure.

Fig. 3 schematically shows a conventional output layer and its output according to an example.

Fig. 4 schematically illustrates two class distributions and the drawbacks of a softmax logistic function according to an example.

Fig. 5 schematically shows an iterative method of training a model according to examples of the present disclosure.

Fig. 6 shows a schematic drawing of an ultrasound system 10 according to examples of the present disclosure.

DESCRIPTION OF THE DRAWINGS

Reference will now be made in detail to examples of the disclosure, examples of which are illustrated in the accompanying drawings. Wherever possible, the same reference numbers will be used throughout the drawings to refer to the same or like parts.

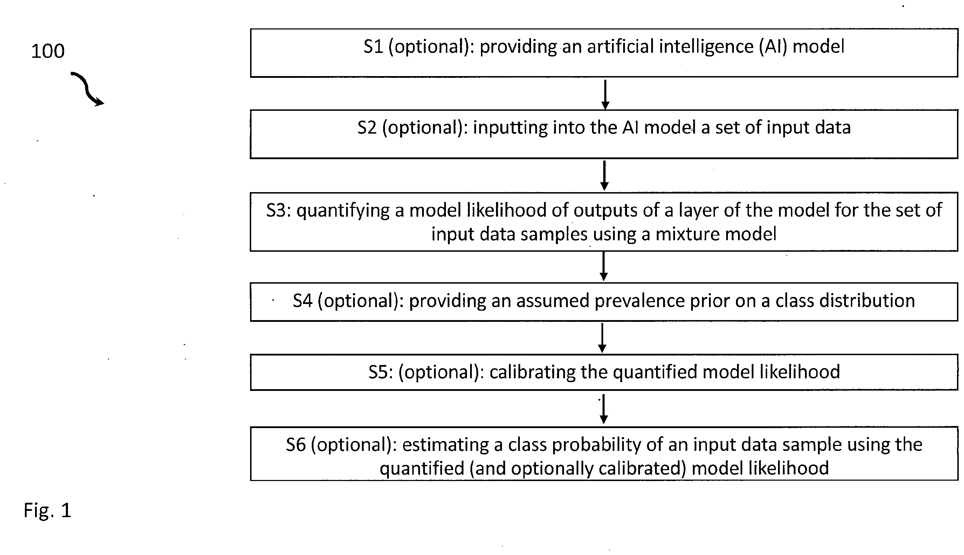

Fig. 1 schematically shows a method of quantifying a posterior confidence according to examples of the present disclosure. The method may comprise a plurality of operations. One or several operations may for example be implemented by a respective software module.

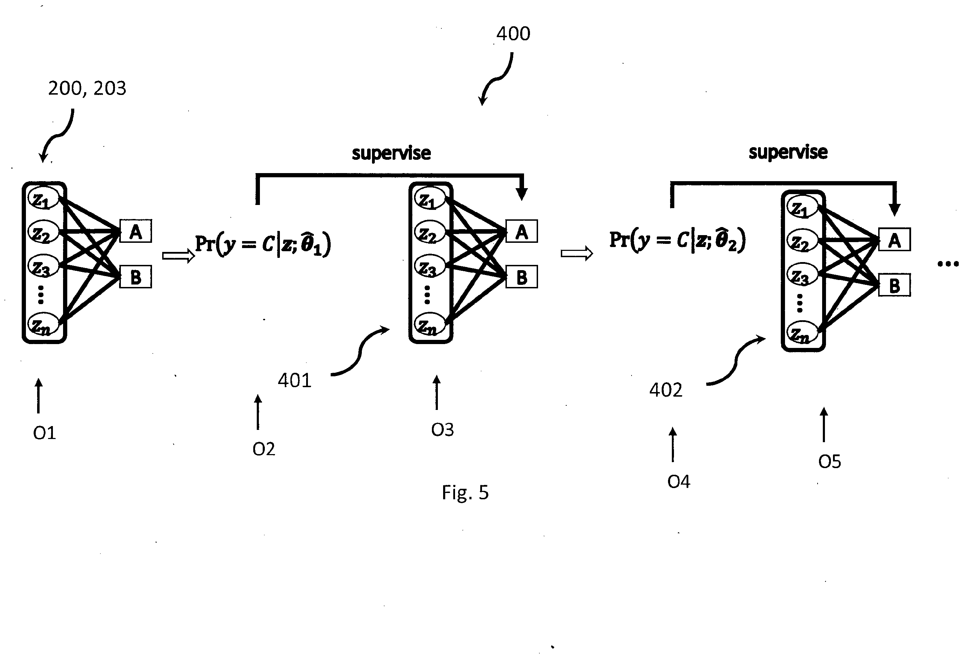

In an optional operation S1, an artificial intelligence (AI) model may be provided. This model may be configured to classify input data. Fig. 5a shows an example of an artificial intelligence (AI) model 200.

In an optional operation S2, a set of input data may be inputted into the AI model. In response, the AI model may estimate respective class probabilities for each sample.

Various classification tasks may be carried out, i.e. various types of class probabilities may be estimated. One example comprises classifying a lesion characteristic of tissue of a patient. For instance, the method may estimate a breast lesion malignancy probability, a Bi-RADS (Breast Imaging Reporting and Data System) lesion classification probability, a liver lesion classification probability, etc.

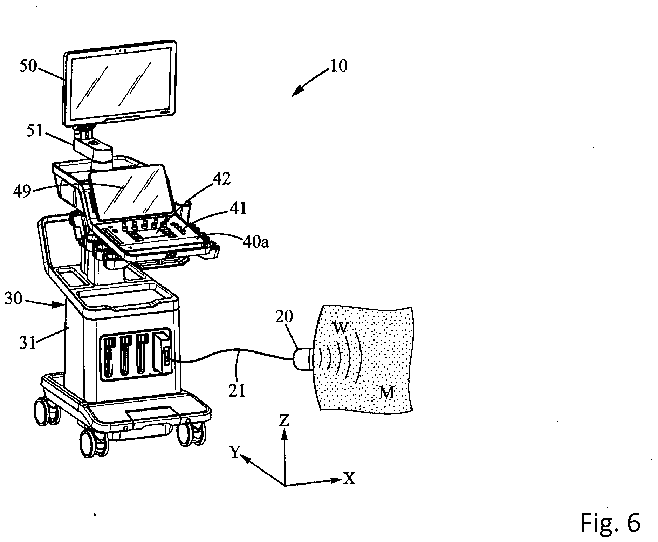

A sample may be for example an image, more particularly for instance a medical image. The image may for example be provided by an imaging system, for instance a medical imaging system. The image may be associated with a medium scanned by the imaging system. For example, the imaging system may comprise at least one of: mammography, tomosynthesis, magnetic resonance imaging (MRI), single-photon emission computerized tomography (SPECT) scan, positron emission tomography (PET) scan, optical coherence tomography (OCCT), optical tomography (OCT), X-ray exam, and ultrasound imaging. An exemplary ultrasound system is shown in fig. 6. In general, the image may be obtained by a first system which performs the method of fig. 1 or the image may be prepared or obtained by a second system and immediately or in a second phase provided to the first system. The transfer may be done via wired or wireless network such as for example 4G/5G, WIFI, LAN, WAN, point to point, etc. In embodiments, the connections may be operable to facility point-to-point communications, connection-oriented communications, connectionless communications, etc.

In an operation S3, a posterior confidence of an output of a layer (for example a hidden layer) of the model is quantified for a set of input data samples using a mixture model. The mixture model may be a probabilistic mixture model and/or a Gaussian mixture model. The operation S3 may comprise recording mean and covariance statistics of the output of the layer for each possible output class of the model.

In an optional operation S4, an assumed prevalence prior on a class distribution may be provided.

In an optional operation S5, the quantified posterior confidence and/or the class probability of the input data sample may be calibrated based on the prevalence prior.

The class distribution may be a known or predetermined class distribution. According, the respective prior data may be provided to the method (for example a system on which the method is running) from an external device, or the data may be stored on the system.

Accordingly, a known class distribution may be used as a prior to calibrate the model and thus increase the accuracy of the model. In one example, the class distribution may concern a distribution of classes (for example Bi-Rads levels) across a population in a particular region. For example, a Bayesian rule may be used for calibrating the quantified posterior confidence and/or the class probability of the input data sample.

Accordingly, the calibration can improve the accuracy of the model output, as it can help the model to better adjust to real world characteristics.

In an optional operation S6, a class probability of an input data sample using the quantified (and optionally calibrated) posterior confidence may be estimated. Said class probability may for example be outputted (for example displayed) to a user.

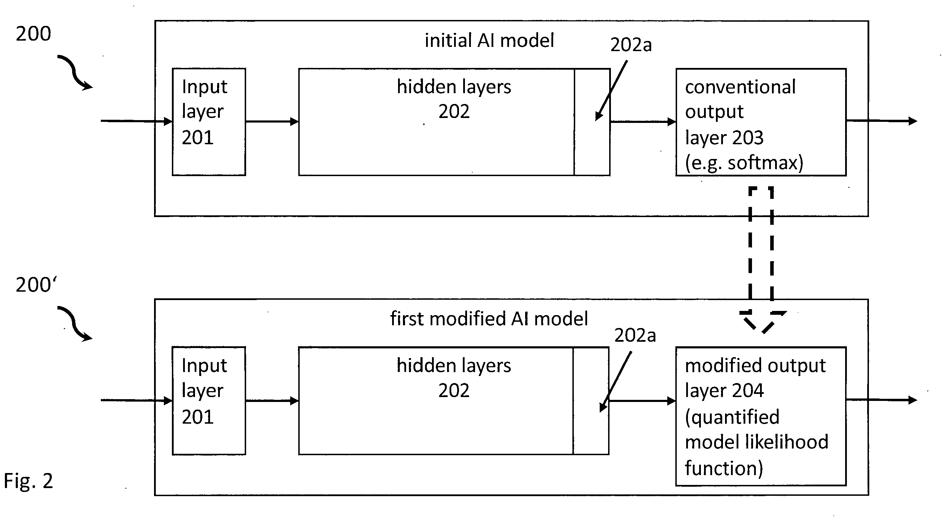

Fig. 2 schematically shows an initial AI model and a first modified AI model according to examples of the present disclosure.

The model 200 may be a first initial model comprising an initial output layer configured to perform a classification task.

The model 200 may be at least one of a machine learning model and a neural network. The model may also be or comprise a deep learning model, for example a neural network, such as a convolutional neural network (CNN).

For example, the model may comprise an optional input layer 201, one or several hidden or intermediate layers 202, and an initial output layer 203. For example, the initial output layer 203 may be configured to perform a K-class softmax rule. K may be at least 2. In other words, the initial output layer may be or may perform or may comprise a softmax logistic function.

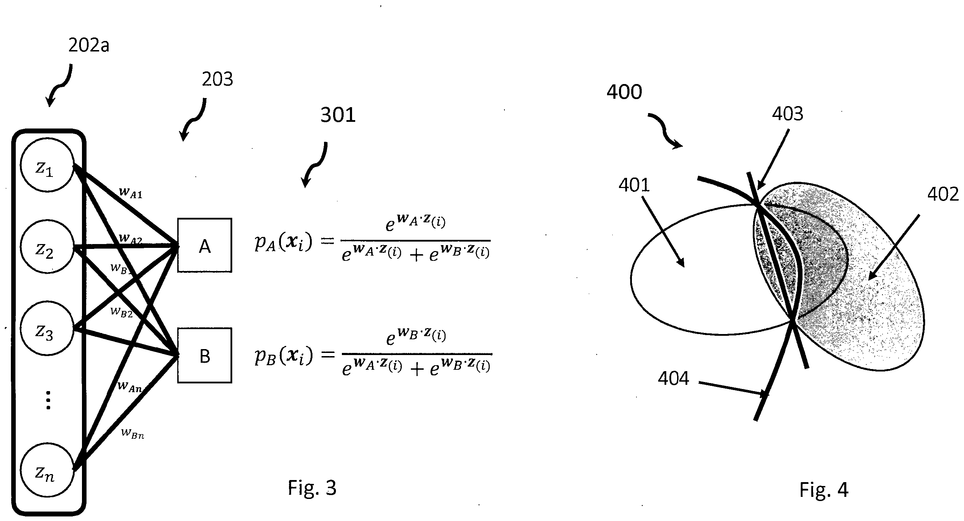

The layer according to the present disclosure may be for example any one of the hidden layers 202. In one example, the layer may be the last or terminating hidden layer z of the model (see also fig. 3) whose output is inputted into the output layer of the model.

With regard to the modified model 200', the first initial model may be modified by replacing the output layer. This modified model 200' may be used in association with a final product, for example a system as shown in fig. 6.

The modified output layer 204 may comprise or use a quantified posterior confidence function to process the data of the terminating hidden layer 202a of the model (cf. also for example fig. 3).

In other words, the "quantified posterior confidence" may be a mathematical function, which for example is able to calculate an output based on an input. Accordingly, the quantified posterior confidence may be used as an output layer of the model 200'. As a consequence, the first modified model 200' does not use the softmax logistic function anymore.

In still other words, the quantified posterior confidence may be expressed as or may constitute a mathematical function. This function may be used as the "new" or replacing output layer 204 which replaces the initial output layer 203 of the model. Thus, the "new" or replacing output layer 204 may receive as input the output of the last hidden layer 202 and calculates on this basis the output of the model 200'. This output may constitute the estimated class probabilities of an input sample provided to the model 200' and fed forward through the model layers.

Advantageously, there is no need to retrain the initial model. It is sufficient to replace the output layer 203, 204, as described above. Hence, the method is computationally significantly less expensive compared to any conventional methods which require training a model.

As a result, the model 200' may be configured to perform a computer-implemented method of estimating a class probability of an input sample comprising according to the present disclosure.

The computer-implemented method of estimating a class probability may be used in or performed by a classification system as described above. The classification system may be part of or associated with for example a medical imaging device. The input sample may be for example a medical image. Various classification tasks may be carried out, i.e. various types of class probabilities may be estimated. One example comprises classifying a lesion characteristic of tissue of a patient. For instance, the method may estimate a breast lesion malignancy probability, a Bi-RADS (Breast Imaging Reporting and Data System) lesion classification probability, a liver lesion classification probability, etc.

However, it is noted that the present disclosure is not restricted to any specific kind of classification task or technical field. Also completely different tasks and technical fields are possible, for example classifying characteristics of a sound wave.

Fig. 3 schematically shows a conventional output layer 203 and its output 301 according to an example. The output layer receives and processes the output of the last hidden layer 202a.

In the Example, the output layer 203 is a K-class softmax logistic function, wherein K=2. In other words, the output layer 203 is configured as a biclassifier estimating the probabilities p of two classes A and B as (cf. eq. (1) and (2)):

$$p_A(x_i) = \frac{e^{w_A \cdot z(i)}}{e^{w_A \cdot z(i)} + e^{w_B \cdot z(i)}} \qquad (1),$$

$$p_B(x_i) = \frac{e^{w_B \cdot z(i)}}{e^{w_A \cdot z(i)} + e^{w_B \cdot z(i)}} \qquad (2),$$

wherein x is the input vector fed to the model,
z is the output vector of the last hidden layer 202a, and
w are the weights attributed to the output of the last hidden layer 202a.

Generally, a K-class softmax logistic function may be defined as, cf. for example eq. (3):

$$p_C(x_i) = \frac{e^{w_C \cdot z(i)}}{\sum_{k=1}^{K} e^{w_k \cdot z(i)}} \qquad (3),$$

wherein $z_{(i)} = [z_1, z_2, \ldots, z_n]_{(i)}^T$ , and
$w_C = [w_{C1}, w_{C2}, \ldots, w_{Cn}]^T$.

However, the softmax function implies several disadvantages. The softmax probability ignores the distribution of the encoded features z. Moreover, the softmax probability ignores the prior probability of the class.

In addition, the softmax probability is adjusted according to its suboptimal piecewise linear decision boundary, as further shown in fig. 4.

Moreover, at least in some cases, the softmax probability seems to be arbitrary.

Fig. 4 schematically shows two class distributions and the drawbacks of a softmax logistic function according to an example 400. In this example, two class distributions 401 and 402 are shown which may partially overlap. The distributions may be gaussian distributions.

A softmax function may be schematically represented by a linear boundary 403, since the softmax is adjusted according to its suboptimal piecewise linear decision boundary. Hence, the softmax does not consider the distribution of the encoded features z. For example, the classification of heteroskedastic features is suboptimal.

In this context, as also shown in the example of fig. 4, two heteroskedastic Gaussians lead to quadratic boundary 404, and not to a linear boundary 403. Accordingly, the softmax does not consider prior probability of class in real world. This means the estimated probability can be likely biased.

In order to overcome these drawbacks, the initial output layer of the model which comprises a softmax logistic function may be advantageously replaced by a modified output layer comprising the quantified posterior confidence function according to the present disclosure.

This quantified posterior confidence function may be determined as follows:

Given a conventional AI model trained by a conventional softmax rule, the model is run through training data (i.e. a set of input data) and record mean and covariance statistics of z for each class C, cf. for example eq. (4) and (5):

$$\mu_z^C = \frac{1}{N_C} \sum_{i \in C} z_{(i)} \qquad (4),$$

$$K_z^C = Cov(\{z_{(i)} | i \in C\}) \qquad (5),$$

wherein

$\mu_z^C$ is a mean statistics,

C is a class (in this example there are two classes A and B),

$K_z^C$ is a covariance statistics.

The likelihood of an input belonging to a class C may be modelled by Gaussian function, cf. for example eq. (6) and (7):

$$p(z|y = C; \widehat{\theta}) = \mathcal{N}(\mu_z^C, K_z^C) \qquad (6),$$

$$\widehat{\theta} = \{\mu_z^A, K_z^A, \mu_z^B, K_z^B\} \qquad (7),$$

wherein

$y$ is the training label, i.e. of an annotated class,
$\mathcal{N}$ is the normal distribution (for example a multi-variate normal distribution),
$\hat{\theta}$ is the set of parameters associated with the multi-variate normal distribution.

Under a uniform prevalence assumption, the posterior confidence may be determined as follows, cf. for example equation eq. (8):

$$\Pr(y = C|z) = \frac{p(z|y=C)}{\sum_c p(z|y = c)} (8).$$

As mentioned before, the quantified posterior confidence and/or the class probability of the input data sample may be calibrated based on a prevalence prior. Given a prior prevalence $P_C = \Pr(y = C)$, the posterior probability that the input belongs to class A may be expressed as, cf. for example eq. (9):

$$\Pr(y = A|z; \widehat{\theta}) = \frac{p(z|y=A;\widehat{\theta})P_A}{\sum_C p(z|y = C; \widehat{\theta})P_C} \qquad (9).$$

Accordingly, the likelihood of x coming from a class C is noted by p(x|C). This may be a Gaussian distribution corresponding to the class C. The Posterior confidence of a class C given x may be: Pr(C|x). This may be derived from a mixture of Gaussian.

Fig. 5 schematically shows an iterative method 400 of training a model according to examples of the present disclosure.

For simplicity, the example only shows a part of the concerned models, i.e. respectively their last hidden layer 203 and their estimated probabilities A, B, similar to the ones used in the example of fig. 3.

In an operation O1, a conventional AI model 200 having an output layer with a softmax logistic function 203 (cf. also fig. 2) may be provided.

In operation O2, the output layer may be replaced by a modified output layer 204 comprising the quantified posterior confidence function according to the present disclosure (cf. also the modified model 200' in fig. 2).

Said modified AI model may be fed with a set of input data samples and may output a respective set of class probabilities. Furthermore, training labels may be generated based on the set of class probabilities.

In an operation O3, an artificial intelligence (AI) model 401 (i.e. which comprises a conventional softmax logistic function) may be trained in a supervised manner using the training labels. For example, the model 401 may be or may correspond to the model 200, which may be trained in operation O3.

In an operation O4 a second artificial intelligence (AI) model may be obtained by replacing the last layer of the trained first artificial intelligence (AI) model (cf. operation O3) by the quantified posterior confidence function. The second artificial intelligence (AI) model may be run to obtain estimated class probabilities for the set of input data samples. The set of input data samples may be re-labelled with the estimated class probabilities.

In an operation O5 a third artificial intelligence (AI) model 402 may be trained in a supervised manner, based on the re-labelled set of input data samples. For example, the model 403 may be or may correspond to the model 200 or the model 401, which may be (re-)trained in operation O5.

In this way, both the labels of training datasets, as well as the trained models may be successively ameliorated to represent the real classification problem more accurately. In particular, a conventional AI model comprising a conventional output layer with a softmax function can be improved.

The method may be iterated one or several times.

In summary, the method according to the present disclosure may comprise one or several of the following operations:

1. Model the network last-layer's output of any given class by a parametric probabilistic model, e.g., a multivariate Gaussian;
2. Assume prevalence prior on class distribution;
3. Formulate the calibrated probability by Bayesian rule;
4. Optionally, reuse the calibrated probability to supervise a model training.

Fig. 6 shows a schematic drawing of an ultrasound system 10 according to examples of the present disclosure.

The ultrasound system 10 may for example be configured to perform a method of the present disclosure. However, any one of thes method according to the present disclosure may also be (at least partially) performed by an external system.

The ultrasound imaging system 10 may comprise:

- a probe 20,
- a processing unit 30 for processing an image (for example a medical image of a patient) on the bases of signals received by the probe,
- a control panel 40a connected to the processing unit, said control panel at least comprising buttons 41 and a touch pad 42, and
- a display 50 for visualizing the image.

The probe 20 may be associated to the processing unit 30 via a cable 21 and/or via wireless connection(s), and it is able to emit ultrasound waves W into a medium M and to receive ultrasound waves W from the medium M, said received ultrasound waves being consequent or resulting from reflections of said emitted ultrasound waves on diffusing particles inside medium M.

The display screen 50 may be a screen for visualizing the image processed by the processing unit 30. The image may be for example a medical image of a patient, for example of a tissue comprising a potential lesion. The display 50 may further indicate an output of the AI model according to the present disclosure, for example an estimated class and optionally a probability of the estimated class. In one example, the class may be a BI-RADS level of a breast lesion. In another example, the class may be a TI-RADS level (Thyroid Imaging Reporting & Data System) of a thyroid, i.e. a gland in the neck. also visualize other information such as scales used in the image, and/or configuration information for the processing or any information such as help information or contextual gesture help for the touch pad 42.

The processing unit 30 may be configured to perform a method according to the present disclosure, for example to perform an AI model of the present disclosure. The processing unit may also send data to an external device for at least partially performing the method of the present disclosure on the external device. Examples of an external device include a server, a computer, a dedicated workstation, a device for displaying images obtained from the electronic control device or any other external device. Accordingly, the method according to the present disclosure may be carried out by at least one of the processing unit or any of the external devices. Furthermore, the process for building the image data based on acquired data, i.e. compiling the ultrasound image data, may be carried out by the same processing device as that for performing the AI model of the present disclosure, or (at least in part) by another one.

According to further examples, the system 10 may include at least one processing unit (or processor) and memory. In examples, the processor and memory unit may be incorporated into the system or may be a computer or computer communicatively linked thereto. Depending on the exact configuration and type of computing device, memory (storing, instructions to evaluate ultrasound data or otherwise perform the methods described herein) may be volatile (such as RAM), nonvolatile (such as RAM, flash memory, etc.), or some combination of the two. Further, the system 10 may also include storage devices (removable and/or non-removable) including, but not limited to, magnetic or optical disks or tape. Similarly, the system 10 may also have input device(s) such as keyboard, mouse, pen, voice input, etc. and/or output device(s) such as a display, speakers, printer, etc. Also included in the environment may be one or more communication connections, such as 4G/5G, WIFI, LAN, WAN, point to point, etc. In embodiments, the connections may be operable to facility point-to-point communications, connection-oriented communications, connectionless communications, etc.

The system 10 may typically include some form of computer readable media.

Communication media embodies computer readable instructions, data structures, program modules, or other data in a modulated data signal such as a carrier wave or other transport mechanism and includes any information delivery media. The term "modulated data signal" means a signal that has one or more of its characteristics set or changed in such a manner as to encode information in the signal. By way of example, and not limitation, communication media includes wired media such as a wired network or direct-wired connection, and wireless media such as acoustic, RF, infrared, microwave, and other wireless media. Combinations of the any of the above should also be included within the scope of computer readable media.

The system 10 may be a single computer operating in a networked environment using logical connections to one or more remote computers. The remote computer may be a personal computer, a server, a router, a network PC, a peer device or other common network node, and typically includes many or all of the elements described above as well as others not so mentioned. The logical connections may include any method supported by available communications media.

Throughout the description, including the claims, the term "comprising a" should be understood as being synonymous with "comprising at least one" unless otherwise stated. In addition, any range set forth in the description, including the claims should be understood as including its end value(s) unless otherwise stated. Specific values for described elements should be understood to be within accepted manufacturing or industry tolerances known to one of skill in the art, and any use of the terms "substantially" and/or "approximately" and/or "generally" should be understood to mean falling within such accepted tolerances.

The terms "record" and "receive" may be used synonymously throughout this disclosure unless denoted differently.

Although the present disclosure herein has been described with reference to particular examples, it is to be understood that these examples are merely illustrative of the principles and applications of the present disclosure.

It is intended that the specification and examples be considered as exemplary only, with a true scope of the disclosure being indicated by the following claims.

A reference herein to a patent document or any other matter identified as prior art, is not to be taken as an admission that the document or other matter was known or that the information it contains was part of the common general knowledge as at the priority date of any of the claims.

## Claims

1. A computer-implemented method, comprising:

   quantifying a posterior confidence of an output of a layer of an artificial intelligence (AI) model for a set of input data samples using a mixture model, the AI model being a classifier model.

2. The method of claim 1, wherein
   the layer is at least one of:

   a hidden layer of the model,
   an intermediate layer of the model,
   a layer other than an output layer of the model, and
   the layer preceding an output layer of the model.

3. The method of claim 1 or 2, further comprising:

   using the quantified posterior confidence as an output layer of the model, and/or
   estimating a class probability of an input data sample using the quantified posterior confidence.

4. The method according to any one of the preceding claims, wherein

   a quantified posterior confidence function is obtained by quantifying a posterior confidence of an output of a layer of the model for a set of input data samples using the mixture model, and/or
   the quantified posterior confidence is a quantified posterior confidence function.

5. The method according to any one of the preceding claims, further comprising:

   providing an assumed prevalence prior on a class distribution,
   calibrating the quantified posterior confidence and/or the class probability of the input data sample based on the prevalence prior.

6. The method of preceding claim, wherein

a Bayesian rule is used for calibrating the quantified posterior confidence and/or the class probability of the input data sample.

7. The method according to any one of the preceding claims, wherein
the mixture model is a probabilistic mixture model and/or a Gaussian mixture model.

8. The method according to any one of the preceding claims, wherein
quantifying the posterior confidence of the layer comprises:

   quantifying the probability distribution of the output of the layer, and/or
   recording mean and covariance statistics of the output of the layer for each possible output class of the model.

9. The method according to any one of the preceding claims, wherein

   the model is at least one of a machine learning model and a neural network, and/or
   the model comprises at least one hidden layer and an output layer.

10. The method according to any one of the preceding claims, wherein

    the model is a first initial model comprising an initial output layer configured to perform a classification task, and/or
    the initial output layer is configured to perform a K-class softmax rule, K being at least two (2).

11. The method according to any one of the preceding claims, further comprising:
    obtaining a first modified model by replacing the initial output layer of the first initial model by the quantified posterior confidence.

12. A computer-implemented method of estimating a class probability of an input sample comprising:
    estimate a class probability for the input sample using the first modified model according to the preceding claim.

13. The method according to the preceding claim, further comprising:

    selecting a prevalence prior as a function of the input sample,
    estimate a calibrated class probability for the input sample based on the prevalence prior using the first modified model according to the preceding claim.

14. A method of generating training labels for an artificial intelligence (AI) algorithm, comprising:

    applying the method of any one of the preceding claims 2 to 13 to the set of input data samples to obtain a set of class probabilities,
    generating the training labels based on the set of class probabilities.

15. A method of training an artificial intelligence (AI) model, comprising:

    performing the method of the preceding claim to generate training labels,
    training a first initial artificial intelligence (AI) model in a supervised manner using the training labels.

16. The method according to the preceding claim, further comprising:

    obtaining a second artificial intelligence (AI) model by replacing the last layer of the trained first artificial intelligence (AI) model by the quantified posterior confidence,
    running the second artificial intelligence (AI) model to obtain estimated class probabilities for the set of input data samples,
    re-labelling the set of input data samples with the estimated class probabilities,
    train a third artificial intelligence (AI) model based on the re-labelled set of input data samples.

17. A computing device, comprising:

    at least one processor, and

at least one memory storing computer-executable instructions, the computer-executable instructions when executed by the processor cause the computing device to perform a method according to any one of the preceding claims.

100

Fig. 1

| S1 (optional): providing an artificial intelligence (AI) model |
| --- |

↓

| S2 (optional): inputting into the AI model a set of input data |
| --- |

↓

| S3: quantifying a model likelihood of outputs of a layer of the model for the set of input data samples using a mixture model |
| --- |

↓

| S4 (optional): providing an assumed prevalence prior on a class distribution |
| --- |

↓

| S5: (optional): calibrating the quantified model likelihood |
| --- |

↓

| S6 (optional): estimating a class probability of an input data sample using the quantified (and optionally calibrated) model likelihood |
| --- |

200

**initial AI model**

| Input layer 201 | hidden layers 202 | 202a | conventional output layer 203 (e.g. softmax) |

200'

**first modified AI model**

| Input layer 201 | hidden layers 202 | 202a | modified output layer 204 (quantified model likelihood function) |

Fig. 2

402

403

404

400

401

**Fig. 4**

301

$$p_A(x_i) = \frac{e^{w_A \cdot z_{(i)}}}{e^{w_A \cdot z_{(i)}} + e^{w_B \cdot z_{(i)}}}$$

$$p_B(x_i) = \frac{e^{w_B \cdot z_{(i)}}}{e^{w_A \cdot z_{(i)}} + e^{w_B \cdot z_{(i)}}}$$

203

202a

A

B

$z_1$  $z_2$  $z_3$  $\cdots$  $z_n$

$w_{A1}$  $w_{A2}$  $w_{B1}$  $w_{B2}$  $w_{An}$  $w_{Bn}$

**Fig. 3**

16

Fig. 5

EP 4 524 816 A1

Fig. 6

Europäisches
Patentamt

European
Patent Office

Office européen
des brevets

# EUROPEAN SEARCH REPORT

**Application Number**

**EP 23 31 5347**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Lee Kimin ET AL: "Robust Inference via Generative Classifiers for Handling Noisy Labels", arXiv.org, 13 May 2019 (2019-05-13), pages 1-17, XP093131256, Ithaca DOI: 10.48550/arxiv.1901.11300 Retrieved from the Internet: URL:https://arxiv.org/pdf/1901.11300.pdf [retrieved on 2024-02-14] * abstract * * Sections 1, 3, and 4 * * Appendices A-C * * Algoritm 1 * | 1-17 | INV. G06N3/045 G06N3/047 G06N3/09 G06N3/096 G06N7/01 |
| A | HOSSEIN MOBAHI ET AL: "Self-Distillation Amplifies Regularization in Hilbert Space", ARXIV.ORG, CORNELL UNIVERSITY LIBRARY, 201 OLIN LIBRARY CORNELL UNIVERSITY ITHACA, NY 14853, 13 February 2020 (2020-02-13), XP081599093, * abstract * * figure 1 * * Section 1 * | 14-16 | |

TECHNICAL FIELDS SEARCHED (IPC)

G06N

-/--

The present search report has been drawn up for all claims

| Place of search | Date of completion of the search | Examiner |
|---|---|---|
| The Hague | 14 February 2024 | Mariani, Giovanni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 2**

Europäisches
Patentamt

European
Patent Office

Office européen
des brevets

## EUROPEAN SEARCH REPORT

Application Number

EP 23 31 5347

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BIRAJA GHOSHAL ET AL: "Estimating uncertainty in deep learning for reporting confidence to clinicians in medical image segmentation and diseases detection", COMPUTATIONAL INTELLIGENCE, BLACKWELL PUBLISHING, HOBOKEN, USA, vol. 37, no. 2, 22 October 2020 (2020-10-22), pages 701-734, XP071705609, ISSN: 0824-7935, DOI: 10.1111/COIN.12411 * abstract * * Sections 1, 3 * ----- | 1-17 | |

TECHNICAL FIELDS
SEARCHED      (IPC)

The present search report has been drawn up for all claims

| Place of search | Date of completion of the search | Examiner |
|---|---|---|
| The Hague | 14 February 2024 | Mariani, Giovanni |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

                    .............................................................................
& : member of the same patent family, corresponding
    document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GOODFELLOW ; IAN ; YOSHUA BENGIO ; AARON COURVILLE.** Deep Learning.. MIT Press, 2016